# EUROPÄISCHE PATENTANMELDUNG

(11) **EP 4 014 862 A1**
(43) Veröffentlichungstag der Anmeldung: **22.06.2022**
(21) Anmeldenummer: 20215642.8
(22) Anmeldetag: 18.12.2020
(51) Int. Cl.: A61B 5/11, A61B 5/00

(54) **VERFAHREN, ÜBERWACHUNGSEINRICHTUNG UND ÜBERWACHUNGSSYSTEM ZUM ERKENNEN EINES EINSTIEGS EINER PERSON IN EIN BETT UND/ODER EINES AUSSTIEGS DER PERSON AUS DEM BETT**

(71) Anmelder: nevisQ GmbH, 52068 Aachen (DE)
(72) Erfinder: KATSANEVAKIS, Stelios, 52066 Aachen (DE); LINK, David Maria, 52074 Aachen (DE); RIRA, Serxhio, 52074 Aachen (DE)
(74) Vertreter: Michalski Hüttermann & Partner Patentanwälte mbB

(57) **Zusammenfassung**

Die Erfindung betrifft ein Verfahren zum Erkennen eines Einstiegs einer Person in ein Bett (10) und/oder eines Ausstiegs einer Person aus dem Bett (10) mittels mindestens einer Überwachungseinrichtung (20) zur Überwachung eines Umfangsbereichs (18) des Bettes (10), wobei die Überwachungseinrichtung (20) zumindest zwei Sensoren (22, 24) zur berührungslosen Objekterfassung in einem Erfassungsbereich (26, 28) des jeweiligen Sensors (22, 24) aufweist und wobei sich die Erfassungsbereiche (26, 28) der Sensoren (22, 24) auf den gemeinsam zu überwachenden Umfangsbereich (18) des Bettes (10) beziehen. Es ist vorgesehen, dass der Einstieg/Ausstieg in dem zu überwachenden Umfangsbereich (18) des Bettes (10) durch Auswertung einer Signalkombination von Sensorsignalen (56, 58) der Sensoren (22, 24) der Überwachungseinrichtung (20) ermittelt wird, wobei bei der Überwachungseinrichtung (20) zumindest ein Sensor (22) der zumindest zwei Sensoren (22, 24) als ein kapazitiver Sensor (22) zur berührungslosen Objekterfassung ausgebildet ist.

Die Erfindung betrifft weiterhin eine entsprechende Überwachungseinrichtung (20) und ein entsprechendes Überwachungssystem mit mehreren Überwachungseinrichtungen (20).

## Beschreibung

Die Erfindung betrifft ein Verfahren zum Erkennen eines Einstiegs einer Person in ein Bett und/oder eines Ausstiegs einer Person aus dem Bett mittels mindestens einer Überwachungseinrichtung zur Überwachung eines Umfangsbereichs des Bettes, wobei die Überwachungseinrichtung zumindest zwei Sensoren zur berührungslosen Objekterfassung in einem Erfassungsbereich des jeweiligen Sensors aufweist und wobei sich die Erfassungsbereiche der Sensoren auf den gemeinsam zu überwachenden Umfangsbereich des Bettes beziehen.

Die Erfindung betrifft weiterhin eine entsprechende Überwachungseinrichtung und ein entsprechendes Überwachungssystem.

Im Bereich der Krankenversorgung und im Pflegebereich gibt es einen erhöhten Bedarf an einer (teil-)automatisierten Überwachung von mehr oder weniger bettlägerigen Personen in ihrem Bett. Dies schließt unter anderem auch ein teilautomatisches/automatisches Erkennen eines Ausstiegs der Person aus dem Bett bzw. Einstiegs der Person in das Bett ein.

Das automatische bzw. teilautomatische Erkennen eines Ein- und/oder Ausstiegs in/aus einem Bett wird durch andere Aktivitäten im Umfangsbereich des Bettes, wie beispielsweise das heraushängen einer Decke oder das Herantreten einer weiteren Person (beispielsweise einer Pflegekraft) an das Bett, erschwert. Je nach Ausgestaltung der Überwachungseinrichtung kann der von ihr überwachte Umfangsbereich des Bettes ein Teil des Gesamtumfangs des Bettes oder der Gesamtumfang des Bettes selbst sein.

Die CN 106994073 A beschreibt ein Verfahren zum Erkennen eines Einstiegs einer Person in ein medizinisches Bett und/oder eines Ausstiegs einer Person aus dem medizinischen Bett mittels einer Überwachungseinrichtung zur Überwachung eines das Bett vollumfänglich umgebenden Umfangsbereichs, wobei die Überwachungseinrichtung vier Sensoren zur berührungslosen Objekterfassung in einem Erfassungsbereich des jeweiligen Sensors aufweist und wobei sich die Erfassungsbereiche der Sensoren auf den gemeinsam zu überwachenden Umfangsbereich des Bettes beziehen. Alle Sensoren der Überwachungseinrichtung sind Infrarot-Sensoren (IR-Sensoren - IR: Infrarot). Jeder dieser Sensoren übernimmt die Überwachung einer (Längs- oder Quer-) Seite des Bettes bzw. Bettgestells, also eines jeweiligen Teilabschnitts des Umfangsbereichs. Mit anderen Worten ergänzen sich die Erfassungsbereiche der einzelnen Sensoren zu dem zu überwachenden Umfangsbereich.

Bei anderen Verfahren zum Erkennen eines Einstiegs einer Person in ein Bett und/oder eines Ausstiegs einer Person aus dem Bett mittels einer Überwachungseinrichtung werden die Sensorsignale unterschiedlicher Sensoren kombiniert und die resultierende Kombination anschließend ausgewertet. Bei der Druckschrift JP 2007020845 A beispielsweise ein Infrarotsensor und ein Drucksensor in der Matratze des Bettes. Dabei beziehen sich die Signale jedoch nicht auf Umfangsbereiche des Bettes.

Es ist die Aufgabe der Erfindung Maßnahmen zum Erkennen eines Einstiegs einer Person in ein Bett und/oder eines Ausstiegs einer Person aus dem Bett bereitzustellen, mittels derer der Ein- und/oder Ausstieg zuverlässig erkannt werden kann/erkannt wird.

Die Lösung der Aufgabe erfolgt erfindungsgemäß durch die Merkmale der unabhängigen Ansprüche. Vorteilhafte Ausgestaltungen der Erfindung sind in den Unteransprüchen angegeben.

Bei dem erfindungsgemäßen Verfahren zum Erkennen eines Einstiegs einer Person in ein Bett und/oder eines Ausstiegs einer Person aus dem Bett mittels mindestens einer Überwachungseinrichtung zur Überwachung eines Umfangsbereichs des Betts, bei der die Überwachungseinrichtung zumindest zwei Sensoren zur berührungslosen Objekterfassung in einem Erfassungsbereich des jeweiligen Sensors aufweist, wobei sich die Erfassungsbereiche der Sensoren auf den gemeinsam zu überwachenden Umfangsbereich des Bettes beziehen, ist vorgesehen, dass der Einstieg/Ausstieg in dem zu überwachenden Umfangsbereich des Bettes durch Auswertung einer Signalkombination von Sensorsignalen der Sensoren der Überwachungseinrichtung ermittelt wird, wobei bei der Überwachungseinrichtung zumindest ein Sensor der zumindest zwei Sensoren als ein kapazitiver Sensor zur berührungslosen Objekterfassung ausgebildet ist. Unter einem kapazitiven Sensor ist ein Sensor zu verstehen, welcher auf Basis der Veränderung der elektrischen Kapazität eines einzelnen Kondensators oder eines Kondensatorsystems arbeitet. Die Beeinflussung der Kapazität durch die zu erfassende Größe kann dabei auf verschiedene Arten erfolgen, die primär durch den Verwendungszweck bestimmt sind. Im vorliegenden Fall ist der Verwendungszweck eine berührungslose Objekterfassung. Der kapazitive Sensor weist dazu beispielsweise eine Elektrode (Messelektrode) und eine RC-Oszillatorschaltung auf. Durch die Annäherung eines Objektes an die Elektrode vergrößert sich die Kapazität und beeinflusst so die Schwingungsamplitude des RC-Oszillators.

Durch die Auswertung der Signalkombination der Sensorsignale mehrerer Sensoren (statt einer Auswertung einzelner Sensorsignale) können Ein- und Ausstieg eindeutiger von anderen typischen Szenen und Aktivitäten im Bereich des Bettumfangs unterschieden werden, insbesondere von einem reinen Herantreten an das Bett, also einen Eintritt in einen Nahbereich vor dem Bett, der jedoch nicht im Zusammenhang mit einem Einstieg in das Bett bzw. Ausstieg aus dem Bett steht. Für die hier vorliegende Anwendung hat sich dabei die Beteiligung mindestens eines kapazitiven Sensors als besonders günstig herausgestellt. Auf diese Weise können aus dem Bett herausragende Objekte oder zusätzliche Objekte im Außenbereich des Bettumfangs gut vom Ein- und Ausstieg einer Person in das Bett/aus dem Bett unterschieden werden.

Dabei müssen die zu kombinierenden Sensorsignale nicht unbedingt von Sensoren unterschiedlichen Sensor-Typs kommen. Auch die Kombination der Sensorsignale von mehreren kapazitiven Sensoren zur berührungslosen Objekterfassung kann bei entsprechender Anordnung zu einer hinreichenden Unterscheidbarkeit von Ein- und Ausstieg einerseits und anderen Szenen und Aktivitäten im Bereich des Bettumfangs andererseits führen. Dazu müssen sich lediglich die "Blickwinkel" der die zu kombinierenden Sensorsignale liefernden kapazitiven Sensoren und/oder deren Erfassungsbereiche unterscheiden.

Gemäß einer bevorzugten Ausführungsform der Erfindung ist zumindest ein weiterer der Sensoren ein Infrarot-Sensor zur berührungslosen Objekterfassung. Die Kombination der Sensorsignale von Infrarot-Sensoren und kapazitiven Sensoren zur berührungslosen Objekterfassung hat sich ebenfalls als günstig zur Unterscheidung von Ein- und Ausstieg einerseits und anderen Szenen und Aktivitäten im Bereich des Bettumfangs andererseits herausgestellt.

Gemäß einer weiteren bevorzugten Ausführungsform der Erfindung ist vorgesehen, dass ein zeitlicher Verlauf der Sensorsignale der Sensoren der Überwachungseinrichtung erstellt wird und bei der Auswertung dieser zeitabhängige Verlauf der Sensorsignale kombiniert und die Signalkombination anschließend analysiert wird. Der zeitabhängige Verlauf der Sensorsignale bei den Signalkombinationen, kann dann unterschiedlichen Szenen und Aktivitäten im Bereich des Bettumfangs zugeordnet werden.

Gemäß noch einer weiteren bevorzugten Ausführungsform der Erfindung überlappen sich die Erfassungsbereiche von zumindest zwei der Sensoren des Sensorsystems, deren Sensorsignale kombiniert werden, zumindest teilweise. Bei dieser Ausführungsform werden beispielsweise die Sensorsignale unterschiedliche Sensorarten kombiniert. Alternativ oder zusätzlich ist mit Vorteil vorgesehen, dass die Erfassungsbereiche von zumindest zwei der Sensoren des Sensorsystems, deren Sensorsignale kombiniert werden, überlappfrei sind.

Bei der erfindungsgemäßen Überwachungseinrichtung zur Überwachung eines Umfangsbereichs eines Bettes um einen Einstieg einer Person in das Bett und/oder einen Ausstieg einer Person aus dem Bett zu erkennen, welche zwei Sensoren zur berührungslosen Objekterfassung in einem Erfassungsbereich des jeweiligen Sensors und eine Auswerteeinheit zur Auswertung von Sensorsignalen der Sensoren aufweist und bei der sich die Erfassungsbereiche der Sensoren auf den gemeinsam zu überwachenden Umfangsbereich des Bettes beziehen lassen, ist vorgesehen, dass die Auswerteeinheit eingerichtet ist, den Einstieg und/oder der Ausstieg durch Auswertung einer Signalkombination von Sensorsignalen der Sensoren zu ermitteln, wobei bei der Überwachungseinrichtung zumindest ein Sensor der zumindest zwei Sensoren als ein kapazitiver Sensor zur berührungslosen Objekterfassung ausgebildet ist. Durch derartige Sensoren und eine derartige Auswerteeinheit können Ein- und Ausstieg in das Bett/aus dem Bett eindeutiger von anderen typischen Szenen und Aktivitäten im Bereich des Bettumfangs unterschieden werden.

Gemäß einer bevorzugten Ausführungsform der erfindungsgemäßen Überwachungseinrichtung ist zumindest ein weiterer Sensor der Sensoren als ein Infrarot-Sensor zur berührungslosen Objekterfassung ausgebildet ist. Es hat sich herausgestellt, dass ein Infrarotsensor eine gute Ergänzung zum kapazitiven Sensor ist.

Gemäß einer weiteren bevorzugten Ausführungsform der erfindungsgemäßen Überwachungseinrichtung ist diese eingerichtet, einen zeitlichen Verlauf der Sensorsignale ihrer Sensoren zu erstellen und bei der Auswertung durch die Auswerteeinheit diesen zeitabhängigen Verlauf der Sensorsignale zu kombinieren und die Kombination anschließend zu analysieren.

Weiterhin ist insbesondere vorgesehen, dass sich die Erfassungsbereiche von zumindest zwei der Sensoren, deren Sensorsignale kombiniert werden, zumindest teilweise überlappen. Alternativ oder zusätzlich ist mit Vorteil vorgesehen, dass die Erfassungsbereiche von zumindest zwei der Sensoren, deren Sensorsignale kombiniert werden, überlappfrei sind.

Gemäß noch einer weiteren bevorzugten Ausführungsform der erfindungsgemäßen Überwachungseinrichtung weist diese eine Rückseite auf, die bei montierter Überwachungseinrichtung dem Bett zugewandt ist und eine gegenüberliegenden Vorderseite, die bei montierter Überwachungseinrichtung dem Bett abgewandt ist.

Dabei ist bevorzugt vorgesehen, dass der zumindest eine kapazitive Sensor zur berührungslosen Objekterfassung eine Elektrode aufweist, wobei
zwischen der Elektrode und der Rückseite der Überwachungseinrichtung eine Abschirmeinrichtung zur Abschirmung der Elektrode gegen rückseitige kapazitive Einflüsse angeordnete ist und/oder
zwischen der Elektrode und der Vorderseite der Überwachungseinrichtung ein Freiraum vorhanden ist. Dieser Freiraum wird dabei insbesondere durch einen Spalt, bevorzugt einen Luftspalt, zwischen der Elektrode und der Vorderseite der Überwachungseinrichtung gebildet.

Das erfindungsgemäße Überwachungssystem zum Erkennen eines Einstiegs einer Person in ein Bett und/oder eines Ausstiegs einer Person aus dem Bett weist schließlich mehrere der vorstehend genannten Überwachungseinrichtungen auf. Gegebenenfalls weist ein solches System weiterhin eine den Überwachungseinrichtungen übergeordnete Steuerungs- und Auswerteeinheit auf, die die Auswertung der internen Auswerteeinheiten der Überwachungseinrichtungen koordiniert und ergänzt.

Nachfolgend wird die Erfindung unter Bezugnahme auf die anliegenden Zeichnungen anhand von bevorzugten Ausführungsbeispielen exemplarisch erläutert, wobei die nachfolgend dargestellten Merkmale sowohl jeweils einzeln als auch in Kombination einen Aspekt der Erfindung darstellen können. Es zeigen:
Fig. 1 eine schematische Darstellung eines Bettes mit einer montierten Überwachungseinrichtung gemäß einer bevorzugten Ausführungsform der Erfindung,
Fig. 2 einen Teil des Bettgestells des Bettes mit Teilen der Überwachungseinrichtung gemäß der bevorzugten Ausführungsform der Erfindung,
Fig. 3 eine Schnittdarstellung der montierten Überwachungseinrichtung gemäß der bevorzugten Ausführungsform der Erfindung,
Fig. 4 eine zeitabhängige Darstellung der Sensorsignale der Sensoren zur berührungslosen Objekterfassung bei einem Ausstieg aus dem Bett.
Fig. 5 eine zeitabhängige Darstellung der Sensorsignale der Sensoren zur berührungslosen Objekterfassung bei einem Einstieg in das Bett.
Fig. 6 eine zeitabhängige Darstellung der Sensorsignale der Sensoren zur berührungslosen Objekterfassung bei einem heraushängen einer Decke aus dem Bett und
Fig. 7 eine zeitabhängige Darstellung der Sensorsignale der Sensoren zur berührungslosen Objekterfassung bei keiner Aktion im überwachten Umfangsbereich des Bettes.

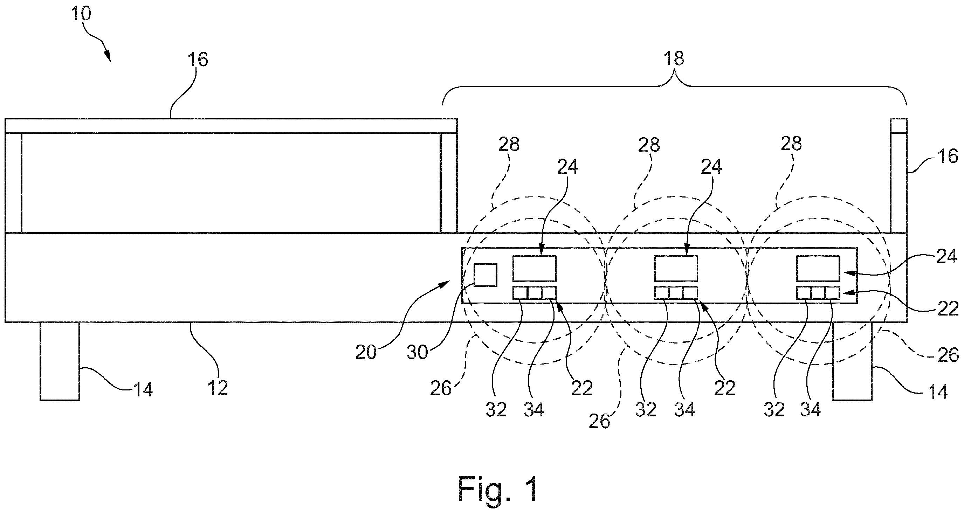

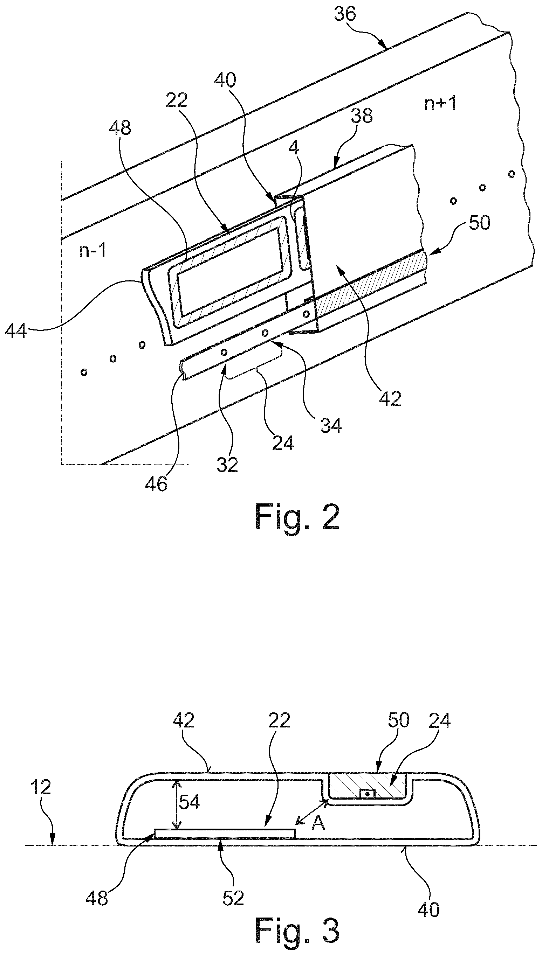

Die Fig. 1 zeigt in schematischer Darstellung ein Bett 10 mit Bettrahmen 12, Füßen 14 und einer die Liegefläche des Bettes 10 in mehreren Bereichen umfänglich begrenzenden Reling 16, die eine auf dem Bett 10 liegende Person vor einem Herausfallen aus dem Bett 10 schützt. Zwischen den Bereichen mit Reling 16 gibt es jedoch mindestens einen Umfangsbereich 18 ohne Reling 16. Nur in diesem Umfangsbereich 18 ist ein Einstieg in das Bett 10 bzw. ein Ausstieg aus dem Bett 10 ohne Schwierigkeiten möglich, da die Reling 16 dies in den anderen Bereichen behindert. Um nun zu erkennen, ob eine Person in diesem Umfangsbereich 18 in das Bett 10 einsteigt oder aus dem Bett 10 aussteigt (das Bett 10 verlässt) ist am Bettrahmen 14 eine Überwachungseinrichtung 20 zum Erkennen eines entsprechenden Einstiegs und/oder Ausstiegs innerhalb des Umfangsbereichs 18 montiert. Die Überwachungseinrichtung 20 weist mehrere (hier im Beispiel sechs) Sensoren 22, 24 zur berührungslosen Objekterfassung in einem jeweiligen Erfassungsbereich 26, 28 der einzelnen Sensoren 22, 24 sowie eine Auswerteeinheit 30 zur Auswertung von Sensorsignalen der Sensoren 22, 24 auf. Die Auswerteeinheit 30 ist dazu mit jedem der Sensoren 22, 24 signaltechnisch verbunden, was im Einzelnen jedoch nicht gezeigt ist. Es gibt zwei Arten (Typen) von Sensoren: kapazitive Sensoren 22 (Cap-Sensoren) mit Erfassungsbereichen 26 und Infrarotsensoren 24 (IR-Sensoren) mit Erfassungsbereichen 28. Jeder der Infrarotsensoren 24 umfasst einen Sender (IR-Sender) 32, der in den entsprechenden Erfassungsbereich 28 hineinstrahlt und einen Empfänger (IR-Empfänger) zum Empfang von Infrarotlicht aus dem Erfassungsbereich 28. Details zu den kapazitiven Sensoren 22 werden im Zusammenhang mit Fig. 2 diskutiert.

Mittels der Überwachungseinrichtung 20 wird der Umfangsbereich 18 überwacht um einen Einstieg einer Person in das Bett 10 und/oder einen Ausstieg einer Person aus dem Bett 10 zu erkennen. Im hier gezeigten Beispiel wird nur ein einzelner Umfangsbereich 18 mittels einer einzigen Überwachungseinrichtung 20 überwacht, da die weiteren umfänglichen Bereiche des Bettes 10 durch die Reling 16 gesichert sind.

Fig. 2 zeigt einen Teil des Bettrahmens 12 des Bettes 10 mit Teilen der am Bettrahmen 12 unterhalb einer Bettkante 36 des Bettes 10 angebrachten bzw. montierten Überwachungseinrichtung 20. Die Überwachungseinrichtung 20 weist neben den im Zusammenhang mit Fig. 1 diskutierten Komponenten 22, 24, 30 auch ein Gehäuse 38 auf, dessen Rückseite 40 dem Bett 10 bzw. Bettrahmen 12 zugewandt ist. Die der Rückseite 40 gegenüberliegende Vorderseite 42 des Gehäuses 38 weist im montierten Zustand der Überwachungseinrichtung 20 dementsprechend vom Bett 10 weg. Da das Gehäuse 38 die weiteren wesentlichen Komponenten 22, 24, 30 der Überwachungseinrichtung 20 einhaust, sind Vorder- und Rückseite 40, 42 des Gehäuses 38 auch Vorder- und Rückseite 40, 42 der Überwachungseinrichtung 20. Die beiden Arten von Sensoren 22, 24, die hier im Beispiel verwendet werden, nämlich kapazitiver Sensor 22 und Infrarotsensor 24, sind in zwei parallel verlaufenden Serienanordnungen auf je einem Träger 44, 46 untereinander angeordnet. Dazu sind hier im Beispiel die kapazitiven Sensoren 22 auf dem einen langgestreckten Träger 44 oberhalb der Infrarotsensoren 24 auf dem anderen langgestreckten Träger 46 angeordnet. Bei dieser Anordnung ist je ein kapazitiver Sensor 22 oberhalb eines der Infrarotsensoren 24 angeordnet. Wesentlicher Bestandteil der kapazitiven Sensoren 22 sind die hier gut sichtbaren Elektroden 48 in Form von Elektrodenschleifen. An der Vorderseite der Überwachungseinrichtung 20 befindet sich vor den Infrarotsensoren 24 ein Infrarot-Fenster 50 für die Infrarotstrahlung der Infrarotsensoren 24.

Die Fig. 3 zeigt eine Schnittdarstellung der montierten Überwachungseinrichtung 20 quer zu der Längserstreckung der Überwachungseinrichtung 20 bzw. der Serienanordnungen ihrer Sensoren 22, 24. Während die Infrarotsensoren 24 hinter dem Infrarot-Fenster 50 nah an der Vorderseite 42 platziert sind, sind die Elektroden 48 der der kapazitiven Sensoren 22 in Richtung der Rückseite 40 platziert. Zwischen den Elektroden 48 und der Vorderseite 40 ist innerhalb des Gehäuses 38 ein Luftspalt, der einen Freiraum 54 zwischen jeweiliger Elektrode 48 und Vorderseite bildet. Zwischen den kapazitiven Sensoren 22 einerseits und den Infrarotsensoren 24 andererseits ist ein gewisser (Mindest-)Abstand A gewährleistet.

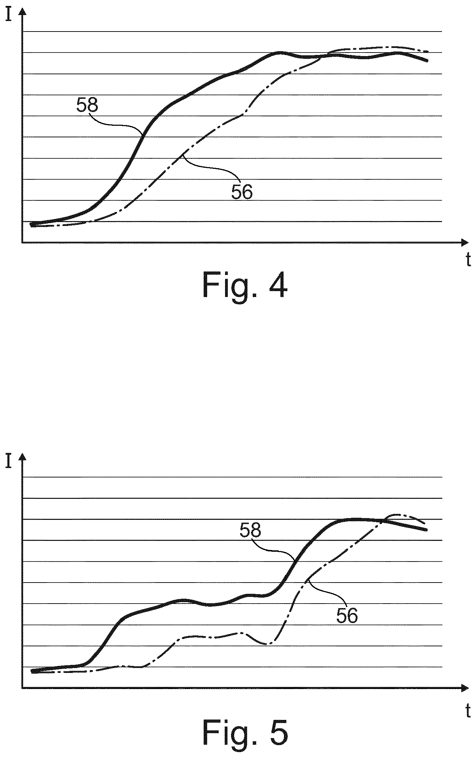

In den Figuren 4 bis 7 sind zeitabhängige Darstellungen der Sensorsignale 56, 58 der Sensoren 22, 24 zur berührungslosen Objekterfassung bei typischen Situationen an der Bettkannte 36 dargestellt.

Um nun den Einstieg/Ausstieg in dem zu überwachenden Umfangsbereich 18 des Bettes 10 zu erkennen wertet die Auswerteeinheit 30 die Signalkombination der Sensorsignale 56 der kapazitiven Sensoren 22 mit den Sensorsignalen 58 der Infrarotsensoren 24 aus. Dies kann im vorliegenden Beispiel paarweise mit den untereinander angeordneten Sensoren 22, 24 unterschiedlicher Sensorart erfolgen oder mit der Summe aller Sensoren 22 der ersten Sensorart (aller kapazitiven Sensoren 22) und der Summe aller Sensoren 24 der zweiten Sensorart (hier: aller Infrarotsensoren 24). Dabei wird ein zeitlicher Verlauf der Sensorsignale 56, 58 der Sensoren 22, 24 erstellt und zur Auswertung dieser zeitabhängige Verlauf der Sensorsignale 56, 58 kombiniert und die Kombination anschließend analysiert. Die Figuren 4 bis 7 zeigen nun ganz typische Muster dieser zeitabhängigen Signalkombinationen.

Die Fig. 4 zeigt eine zeitabhängige Darstellung der Sensorsignale 56, 58 der Sensoren 22, 24 zur berührungslosen Objekterfassung bei einem Ausstieg einer Person aus dem Bett 10. Dabei kommt es erst zu einem kontinuierlichen Anstieg des Sensorsignals 58 der Infrarotsensoren 24, zeitlich versetzt gefolgt von einem ähnlichen kontinuierlichen Anstieg des Sensorsignals 56 der kapazitiven Sensoren 22 bis hin zu einem maximalen Sensorsignal.

Demgegenüber zeigt die Fig. 5 eine zeitabhängige Darstellung der Sensorsignale 56, 58 der Sensoren 22, 24 zur berührungslosen Objekterfassung bei einem Einstieg in das Bett 10. Hier kommt es erst zu einem zweistufigen Anstieg des Sensorsignals 58 der Infrarotsensoren 24 zeitlich versetzt gefolgt von einem ähnlichen zweistufigen Anstieg des Sensorsignals 56 der kapazitiven Sensoren 22 bis hin zu einem maximalen Sensorsignal.

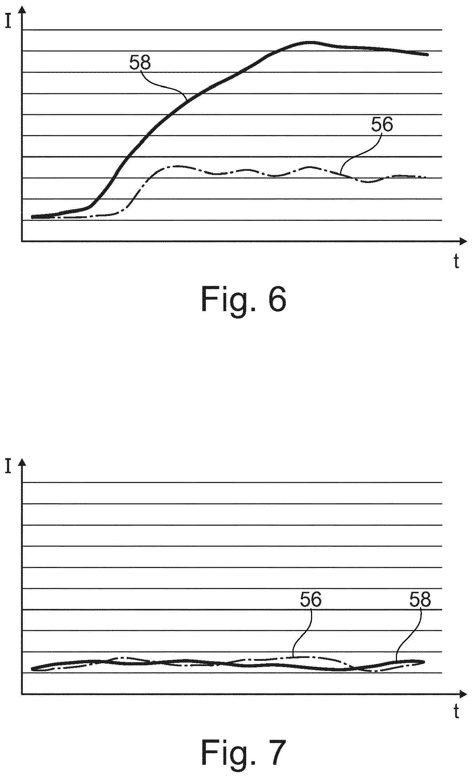

Die Fig. 6 zeigt eine zeitabhängige Darstellung der Sensorsignale 56, 58 der Sensoren 22, 24 zur berührungslosen Objekterfassung bei einem Heraushängen einer (nicht gezeigten) Decke aus dem Bett 10. Hierbei kommt es erst zu einem kontinuierlichen Anstieg des Sensorsignals 58 der Infrarotsensoren 24, zeitlich versetzt gefolgt von einem kurzen und deutlich schwächeren Anstieg des Sensorsignals 56 der kapazitiven Sensoren 22. Der Anstieg des Sensorsignals 56 der kapazitiven Sensoren 22 ist vor allem wegen des Luftspalts bzw. Freiraums 54 so deutlich schwächer.

Schließlich zeigt Fig. 7 eine zeitabhängige Darstellung der Sensorsignale 56, 58 der Sensoren 22, 24 zur berührungslosen Objekterfassung, wenn keinerlei Aktion im überwachten Umfangsbereich 18 des Bettes 10 vorliegt. Hier bleiben die Sensorsignale 56, 58 relativ konstant auf niedrigem Niveau.

Im Folgenden sollen wesentliche Parameter mit Bezug auf das in den Figuren beschriebene Ausführungsbeispiel noch einmal mit anderen Worten diskutiert werden:
Für die Abtastraten der Sensoren 22, 24 ergeben sich folgende geeignete Werte:
Für die Infrarot-Sensoren 24 fünf Messungen pro Sekunde und für die kapazitiven Sensoren 22 (Cap-Sensoren) fünfzig Messungen pro Sekunde.

Die Auswertung der Signalkombinationen der zeitabhängigen Sensorsignale 56, 58 der Sensoren 22, 24 ermöglicht ein vergleichsweise sicheres Erkennen eines Einstiegs in das Bett 10 bzw. Ausstiegs aus dem Bett 10 und schließt Fehlalarme - beispielsweise durch Personen, die sich dem Bett 10 nur nähern - weitgehend aus.

Aus Fig. 5 (Ausstieg aus dem Bett 10) ist ersichtlich, dass das weitreichendere IR-Signal 58 dem kurzreichweitigeren CAP-Signal 56 vorausgeht.

Die Fig. 6 zeigt ein starkes IR-Signal 58 durch optische Verdeckung, aber nur geringfügige Erhöhung des CAP-Signals 56 (diese Erhöhung ist signifikant geringer, als wenn eine Person aus dem Bett 10 aussteigen würde - vgl. Fig. 4). Der Luftspalt bzw. der Freiraum 54 lässt das CAP-Signal 56 dabei nicht allzu hoch steigen. Durch die Signalkombination wird hier also sichergestellt, dass verdeckende Objekte -wie Decken oder Kleidung - keinen Fehlalarm auslösen.

Der diagonale Abstand A von Elektrode 48 und IR-Sensor 24 minimiert die parasitäre Kapazität verursacht durch die IR-Sensorik, sodass die Baseline vom Cap-Sensor 22 nur vernachlässigbar erhöht wird.

### Bezugszeichen

- 10: Bett
- 12: Bettrahmen
- 14: Fuß (Bett)
- 16: Reling
- 18: Umfangsbereich
- 20: Überwachungseinrichtung
- 22: kapazitiver Sensor
- 24: Infrarotsensor
- 26: Erfassungsbereich (kapazitiver Sensor)
- 28: Erfassungsbereich (Infrarotsensor)
- 30: Auswerteeinheit
- 32: Sender
- 34: Empfänger
- 36: Bettkante
- 38: Gehäuse
- 40: Rückseite
- 42: Vorderseite
- 44: Träger
- 46: Träger
- 48: Elektrode
- 50: Infrarot-Fenster
- 52: Abschirmeinrichtung
- 54: Freiraum
- 56: Sensorsignal des kapazitiven Sensors
- 58: Sensorsignal des Infrarotsensors
- A: Abstand (kapazitiver Sensor - Infrarotsensor)

## Patentansprüche

1. Verfahren zum Erkennen eines Einstiegs einer Person in ein Bett (10) und/oder eines Ausstiegs einer Person aus dem Bett (10) mittels mindestens einer Überwachungseinrichtung (20) zur Überwachung eines Umfangsbereichs (18) des Bettes (10), wobei die Überwachungseinrichtung (20) zumindest zwei Sensoren (22, 24) zur berührungslosen Objekterfassung in einem Erfassungsbereich (26, 28) des jeweiligen Sensors (22, 24) aufweist und wobei sich die Erfassungsbereiche (26, 28) der Sensoren (22, 24) auf den gemeinsam zu überwachenden Umfangsbereich (18) des Bettes (10) beziehen,
**dadurch gekennzeichnet, dass**
der Einstieg/Ausstieg in dem zu überwachenden Umfangsbereich (18) des Bettes (10) durch Auswertung einer Signalkombination von Sensorsignalen (56, 58) der Sensoren (22, 24) der Überwachungseinrichtung (20) ermittelt wird, wobei bei der Überwachungseinrichtung (20) zumindest ein Sensor (22) der zumindest zwei Sensoren (22, 24) als ein kapazitiver Sensor (22) zur berührungslosen Objekterfassung ausgebildet ist.

2. Verfahren nach Anspruch 1, wobei bei der Überwachungseinrichtung (20) zumindest ein weiterer Sensor (24) der Sensoren (22,24) als ein Infrarot-Sensor (24) zur berührungslosen Objekterfassung ausgebildet ist.

3. Verfahren nach Anspruch 1 oder 2, wobei ein zeitlicher Verlauf der Sensorsignale (56, 58) der Sensoren (22, 24) der Überwachungseinrichtung (20) erstellt wird und bei der Auswertung dieser zeitabhängige Verlauf der Sensorsignale (56, 58) kombiniert und die Kombination anschließend analysiert wird.

4. Verfahren nach einem der Ansprüche 1 bis 3, wobei sich die Erfassungsbereiche (26, 28) von zumindest zwei der Sensoren (22, 24) des Sensorsystems (20), deren Sensorsignale (56, 58) kombiniert werden, zumindest teilweise überlappen.

5. Verfahren nach einem der Ansprüche 1 bis 4, wobei die Erfassungsbereiche (26, 28) von zumindest zwei der Sensoren (22, 24) des Sensorsystems (20), deren Sensorsignale (56, 58) kombiniert werden, überlappfrei sind.

6. Überwachungseinrichtung (20) zur Überwachung eines Umfangsbereichs (18) eines Bettes (10) um einen Einstieg einer Person in das Bett (10) und/oder einen Ausstieg einer Person aus dem Bett (10) zu erkennen, mit zwei Sensoren (22, 24) zur berührungslosen Objekterfassung in einem Erfassungsbereich (26, 28) des jeweiligen Sensors (22, 24) und einer Auswerteeinheit (30) zur Auswertung von Sensorsignalen (56, 58) der Sensoren (22, 24), wobei sich die Erfassungsbereiche (26, 28) der Sensoren (22, 24) auf den gemeinsam zu überwachenden Umfangsbereich (18) des Bettes (10) beziehen lassen,
**dadurch gekennzeichnet, dass**
die Auswerteeinheit (30) eingerichtet ist, den Einstieg und/oder der Ausstieg durch Auswertung einer Signalkombination von Sensorsignalen (56, 58) der Sensoren (22, 24) zu ermitteln, wobei bei der Überwachungseinrichtung (20) zumindest ein Sensor (22) der zumindest zwei Sensoren (22, 24) als ein kapazitiver Sensor (22) zur berührungslosen Objekterfassung ausgebildet ist.

7. Überwachungseinrichtung nach Anspruch 6, wobei zumindest ein weiterer Sensor (24) der Sensoren (22,24) als ein Infrarot-Sensor (24) zur berührungslosen Objekterfassung ausgebildet ist.

8. Überwachungseinrichtung nach Anspruch 6 oder 7, wobei diese eingerichtet ist, einen zeitlichen Verlauf der Sensorsignale (56, 58) ihrer Sensoren (22, 24) zu erstellen und bei der Auswertung durch die Auswerteeinheit (30) diesen zeitabhängigen Verlauf der Sensorsignale (56, 58) zu kombinieren und die Kombination anschließend zu analysieren.

9. Überwachungseinrichtung nach einem der Ansprüche 6 bis 8, wobei sich die Erfassungsbereiche (26, 28) von zumindest zwei der Sensoren (22, 24), deren Sensorsignale (56, 58) kombiniert werden, zumindest teilweise überlappen.

10. Überwachungseinrichtung nach einem der Ansprüche 6 bis 9, wobei die Erfassungsbereiche (26, 28) von zumindest zwei der Sensoren (22, 24), deren Sensorsignale (56, 58) kombiniert werden, überlappfrei sind.

11. Überwachungseinrichtung nach einem der Ansprüche 6 bis 10, mit einer Rückseite (40), die bei montierter Überwachungseinrichtung dem Bett (10) zugewandt ist und einer gegenüberliegenden Vorderseite (42), die bei montierter Überwachungseinrichtung dem Bett (10) abgewandt ist.

12. Überwachungseinrichtung nach Anspruch 11, wobei der zumindest eine kapazitive Sensor (22) zur berührungslosen Objekterfassung eine Elektrode (48) aufweist und wobei,
- zwischen der Elektrode (48) und der Rückseite (40) der Überwachungseinrichtung (20) eine Abschirmeinrichtung (52) zur Abschirmung der Elektrode (46) gegen rückseitige kapazitive Einflüsse angeordnete ist und/oder
- zwischen der Elektrode (48) und der Vorderseite (42) der Überwachungseinrichtung (20) ein Freiraum (54) vorhanden ist.

13. Überwachungssystem zum Erkennen eines Einstiegs einer Person in ein Bett (10) und/oder eines Ausstiegs einer Person aus dem Bett (10) mit mehreren Überwachungseinrichtungen (20) nach einem der Ansprüche 6 bis 12.
